# EUROPÄISCHE PATENTSCHRIFT

(11) **EP 3 021 755 B1**
(45) Veröffentlichungstag und Bekanntmachung des Hinweises auf die Patenterteilung: **25.08.2021**
(21) Anmeldenummer: 14750155.5
(22) Anmeldetag: 15.07.2014
(51) Int. Cl.: A61B 6/14

(54) **VERFAHREN ZUR ERZEUGUNG EINER RÖNTGENAUFNAHME**
METHOD FOR PRODUCING AN X-RAY IMAGE
PROCÉDÉ POUR LA PRODUCTION D'UNE RADIOGRAPHIE

(30) Priorität: 15.07.2013 DE 102013213761
(43) Veröffentlichungstag der Anmeldung: 25.05.2016
(73) Patentinhaber: Sirona Dental Systems GmbH, 64625 Bensheim (DE)
(72) Erfinder: ABKAI, Ciamak, 68542 Heddesheim (DE); LINDENBERG, Kai, 64395 Wersau (DE)
(74) Vertreter: Özer, Alpdeniz
(86) Internationale Anmeldenummer: PCT/EP2014/065091
(87) Internationale Veröffentlichungsnummer: WO 2015/007710

(56) Entgegenhaltungen:
- EP-A1- 1 815 794
- EP-A1- 2 130 491
- DE-A1-102008 035 412
- None

## Beschreibung

### Technisches Gebiet

Die Erfindung betrifft ein Verfahren zur Erzeugung einer Röntgenaufnahme eines Objekts mittels einer Röntgenvorrichtung, wobei während eines zumindest teilweisen Umlaufs eine Röntgenquelle und einen Röntgendetektor um ein Objekt bewegt werden, wobei die von der Röntgenquelle erzeugte und das Objekt durchlaufende Röntgenstrahlen aus mehreren verschiedenen Richtungen vom Röntgendetektor erfasst werden.

### Stand der Technik

Aus dem Stand der Technik sind mehrere Verfahren zur Erzeugung von Röntgenaufnahmen bekannt, wobei eine Röntgenquelle und ein Röntgendetektor um ein Objekt bewegt werden. Während eines Umlaufs werden aus verschiedenen Richtungen zweidimensionale Röntgenaufnahmen des Objekts erzeugt und anschließend aus den zweidimensionalen Röntgenaufnahmen eine dreidimensionale Röntgenaufnahme oder eine Panoramaschichtaufnahme des Objekts durch ein Rekonstruktionsverfahren berechnet.

In der EP 2146321 A1 ist eine Röntgenvorrichtung mit einer Röntgenquelle und einem Röntgendetektor offenbart, die um ein Objekt bewegt werden, wobei das Objekt mittels einer Kamera aufgenommen wird. Dabei ist die Kamera außerhalb der Röntgenvorrichtung angeordnet und wird nicht mit gedreht. Die optischen Aufnahmen der Kamera des Objekts werden dann verwendet, um Bewegungen des Objekts während der Röntgenaufnahme zu kompensieren.

Ein Nachteil dieses Verfahrens besteht darin, dass während der Aufnahme eine Deformation des Tragarms oder eine Bewegungstrajektorie des Objekts relativ zum Tragarm zu Abbildungsfehlern führen kann. Zur Korrektur dieser Abbildungsfehler ist ein Verfahren bekannt, wobei eine Kalibrierung der Röntgenvorrichtung mittels eines Kalibrierungsphantoms vor jeder Röntgenaufnahme durchgeführt wird. Dadurch werden Veränderungen der Umlauftrajektorie ermittelt, die beispielsweise durch mechanische Deformationen des Tragarms oder der verwendeten Lager verursacht werden können.

Die Aufgabe der vorliegenden Erfindung besteht also darin, ein Verfahren zur Erzeugung einer Röntgenaufnahme bereitzustellen, das auf eine einfache Art und Weise eine fehlerfreie dreidimensionale Röntgenaufnahme ohne vorangehende Kalibrierungsmessungen ermöglicht.

### Darstellung der Erfindung

Die Erfindung betrifft ein Verfahren zur Erzeugung einer Röntgenaufnahme eines Objekts mittels einer Röntgenvorrichtung, wobei während eines zumindest teilweisen Umlaufs eine Röntgenquelle und ein Röntgendetektor um ein Objekt bewegt werden, wobei die von der Röntgenquelle erzeugte und das Objekt durchlaufende Röntgenstrahlen aus mehreren verschiedenen Richtungen mittels des Röntgendetektors erfasst werden. Dabei nimmt mindestens eine optische Kamera während des Umlaufs das Objekt auf und mindestens eine erste optische Aufnahme wird erzeugt, wobei die optische Kamera eine bekannte und definierte Lagebeziehung zur Röntgenquelle und/oder zum Röntgendetektor aufweist. Die optische Aufnahme wird dann anschließend verwendet, um eine Bewegungstrajektorie des Objekts relativ zur Röntgenquelle und/oder zum Röntgendetektor zu bestimmen.

Das Röntgenaufnahmeverfahren kann entweder ein dreidimensionales Röntgenaufnahmeverfahren, wie DVT oder CT, oder ein zweidimensionales Röntgenaufnahmeverfahren, wie eine Panoramaschichtaufnahme, sein.

Bei dem sogenannten digitalen Volumentomographieverfahren (DVT) oder bei der sogenannten Computertomographie (CT) werden die Röntgenquelle und der Röntgendetektor um das Objekt, wie einen Patientenkopf, bewegt, wobei aus verschiedenen Richtungen zweidimensionale Projektionsaufnahmen erzeugt werden, wobei im nächsten Schritt aus diesen Projektionsaufnahmen ein dreidimensionales Volumen berechnet wird. Diese berechnete dreidimensionale Röntgenaufnahme kann dann in einer Benutzersoftware mittels einer Anzeigevorrichtung, wie eines Bildschirms, dargestellt werden. Die Bildgebung beruht also auf einer kontinuierlichen Aufnahme der Projektion aus unterschiedlichen Richtungen, wobei unter Verwendung eines Rekonstruktionsverfahrens die dreidimensionale Röntgenaufnahme berechnet wird, wobei den sogenannten Voxeln der dreidimensionalen Röntgenaufnahme die jeweiligen Röntgenabsorptionswerte zugeordnet werden. Bei einer Panoramaschichtaufnahme werden die Röntgenquelle und der Röntgendetektor ebenfalls um das Objekt bewegt, wobei eine scharfe Schicht mit einem Verwischungsanteil ausgebildet wird. Durch die Veränderung der Umlauftrajektorie der Röntgenquelle und des Röntgendetektors kann die scharfe Schicht entsprechend an das aufzunehmende Objekt angepasst werden.

Die Röntgenquelle und der Röntgendetektor sind an einem Tragarm angebracht. Der Tragarm kann Lager und Antriebsmittel aufweisen, wobei Antriebsmittel, wie ein Elektromotor, so angesteuert werden kann, dass die Röntgenquelle und der Röntgendetektor entlang der geplanten Trajektorie um das Objekt bewegt werden. Bei einem DVT-Verfahren oder einem CT-Verfahren kann die Trajektorie der Röntgenquelle und des Röntgendetektors kreisförmig sein.

Die optische Kamera kann eine beliebige digitale Videokamera sein, die das Objekt kontinuierlich während des gesamten Umlaufs aufnimmt. Die optische Kamera weist eine definierte Lagebeziehung zur Röntgenquelle und/oder zum Röntgendetektor auf, so dass die Deformationen des Tragarms und/oder eine relative Bewegungen des Objekts bestimmt werden können. Die Deformation des Tragarms kann beispielsweise durch eine externe Belastung des Tragarms oder durch Vibrationen erzeugt werden, die durch Antriebsmittel des Tragarms während des Betriebs verursacht werden.

Die optische Kamera weist eine bekannte und definierte Lagebeziehung zur Röntgenquelle und/oder zum Röntgendetektor auf. Damit ist die optische Kamera am Röntgendetektor angebracht. Die Lage und Orientierung der optischen Kamera relativ zur Röntgenquelle und/oder zum Röntgendetektor ist damit zu jedem Zeitpunktwährend der Vermessung bekannt.

Die Bewegungstrajektorie des Objekts definiert also die zeitabhängige Lage und Orientierung des Objekts relativ zur Röntgenquelle und/oder zum Röntgendetektor während des Umlaufs.

Das Objekt, wie ein Patientenkopf, muss nicht fixiert werden, sondern kann während des Umlaufs relativ zum Tragarm frei innerhalb eines Aufnahmevolumens der Röntgenvorrichtung bewegt werden.

Ein Vorteil dieses Verfahrens besteht darin, dass eine Kalibrierung mittels eines Kalibrierungsphantoms vor jeder Messung nicht mehr notwendig ist, da die Ermittlung der relativen Bewegungstrajektorie unmittelbar während der Vermessung mittels der optischen Kamera erfolgt. Damit wird also eine so genannte Online-Kalibrierung während der Vermessung durchgeführt.

Ein weiterer Vorteil dieses Verfahrens ist, dass das Objekt, wie ein Patientenkopf, nicht wie bei herkömmlichen Verfahren unter Verwendung von Fixiermitteln, wie eines Aufbissstücks und/oder einer Auflagefläche, relativ zur Röntgenvorrichtung fixiert werden muss. Der Patient kann sich also innerhalb festgelegter Toleranzgrenzen innerhalb des Aufnahmevolumens frei bewegen.

Bei der Berechnung bzw. Rekonstruktion der dreidimensionalen Röntgenaufnahme aus den einzelnen zweidimensionalen Röntgenaufnahmen werden also die Deformation des Tragarms und die Bewegungstrajektorie des Objekts relativ zur Röntgenquelle und/oder zum Röntgendetektor berücksichtigt.

Dabei kann die mindestens eine optische Aufnahme verwendet wird, um Bewegungen des Objektes relativ zur Röntgenquelle und/oder Röntgendetektor während des Umlaufs zu bestimmen.

Damit wird also die Bewegungstrajektorie des Objekts bestimmt.

Die Röntgenquelle und der Röntgendetektor sind fest an einem Tragarm angebracht, wobei die optische Aufnahme verwendet wird, um zusätzlich eine Deformation des Tragarms während des Umlaufs zu bestimmen. Dadurch werden bei der Berechnung bzw. Rekonstruktion der dreidimensionalen Röntgenaufnahme die Deformation des Tragarms und die Bewegungstrajektorie des Objekts relativ zur Röntgenquelle und/oder zum Röntgendetektor berücksichtigt, so dass mögliche Abbildungsfehler minimiert werden können.

Durch die feste Anbringung der optischen Kamera am Röntgendetektor ist die Lage der optischen Kamera relativ zur Röntgenquelle, zum Röntgendetektor und/oder zum Tragarm festgelegt.

Vorteilhafterweise kann die ermittelte Bewegungstrajektorie des Objekts relativ zur Röntgenquelle und/oder zum Röntgendetektor verwendet wird, um eine dreidimensionale Röntgenaufnahme aus mehreren zweidimensionalen Röntgenaufnahmen, die aus den unterschiedlichen Richtungen mittels des Röntgendetektors erfasst wurden, zu berechnen.

Dadurch wird unter Verwendung des Rekonstruktionsverfahrens eine fehlerfreie dreidimensionale Röntgenaufnahme berechnet, die die tatsächliche ermittelte Bewegungstrajektorie und nicht nur eine geplante oder kalibrierte abweichende Bewegungstrajektorie, wie bei herkömmlichen CT-Röntgenvorrichtungen, berücksichtigt.

Vorteilhafterweise kann zusätzlich zur ersten optischen Kamera eine zweite optische Kamera während des Umlaufs das Objekts aufnehmen, wobei eine zweite optische Aufnahme des Objekts erzeugt wird, wobei beide optischen Kameras eine bekannte und definierte Lagebeziehung zueinander aufweisen.

Die zweite optische Kamera kann eine beliebige optische digitale Videokamera sein, die das Objekt aus einer zweiten Aufnahmerichtung während des Umlaufs aufnimmt. Dadurch wird die Bestimmung der Lage des Objekts relativ zum Tragarm verbessert.

Vorteilhafterweise können am Objekt optische Markierungen angebracht sein, die mittels der optischen Kamera erfasst werden können.

Die optischen Markierungen können beispielsweise optisch eindeutig erkennbare Strukturen aufweisen. Die optischen Markierungen können einen Klebefilm aufweisen, so dass sie am Objekt, wie einem Patientenkopf, angebracht werden können.

Die optischen Markierungen werden dann in den optischen Aufnahmen der optischen Kameras erkannt, so dass die Lageänderung des Objekts während des gesamten Umlaufs relativ zur Röntgenquelle und/oder zum Röntgendetektor bestimmt werden kann.

Vorteilhafterweise können markante Strukturen des Objekts, wie das Ohr, die Lippen, das Kinn oder die Augen eines Patientenkopfes, erfasst werden, um die Lagebeziehung des Objekts relativ zum Tragarm zu bestimmen.

Dadurch kann die Lageänderung des Objekts während des Umlaufs anhand der markanten Strukturen des Objekts mitverfolgt werden.

Vorteilhafterweise können die optischen Markierungen am Objekt und /oder die markanten Strukturen des Objekts unter Anwendung von Mustererkennungsalgorithmen in der optischen Aufnahme erkannt werden.

Bei der Verwendung von Mustererkennungsalgorithmen werden übereinstimmende Bereiche in den optischen Videoaufnahmen ermittelt und zur Bestimmung der Lageänderung des Objekts verwendet. Die übereinstimmenden Bereiche können markante Strukturen, wie das Ohr, die Lippen, das Kinn oder die Augen des Patientenkopfes sein.

Vorteilhafterweise kann eine Korrektur von Abbildungsfehlern dadurch erfolgt, dass bei der Berechnung der dreidimensionalen Röntgenaufnahme eine geplante Soll-Trajektorie des Umlaufs durch die mittels der optischen Kamera ermittelte tatsächliche Bewegungstrajektorie ersetzt wird, wobei die tatsächliche Bewegungstrajektorie die Deformation des Tragarms und/oder die relative Bewegungstrajektorie des Objekts während des Umlaufs berücksichtigt.

Dadurch wird in der Software zur Berechnung bzw. zur Rekonstruktion des dreidimensionalen Röntgenbildes die geplante Soll-Trajektorie durch die mittels der optischen Kamera gemessene Ist-Trajektorie ersetzt. Auf diese Weise kann nach dem vorliegenden Verfahren eine herkömmliche Röntgenvorrichtung einfach modifiziert werden, indem mindestens eine optische Kamera an der Röntgenquelle, am Röntgendetektor und/oder am Tragarm angebracht wird und lediglich die Bewegungstrajektorie in der bereits vorhandenen Software ersetzt wird.

### Kurzbeschreibung der Zeichnungen

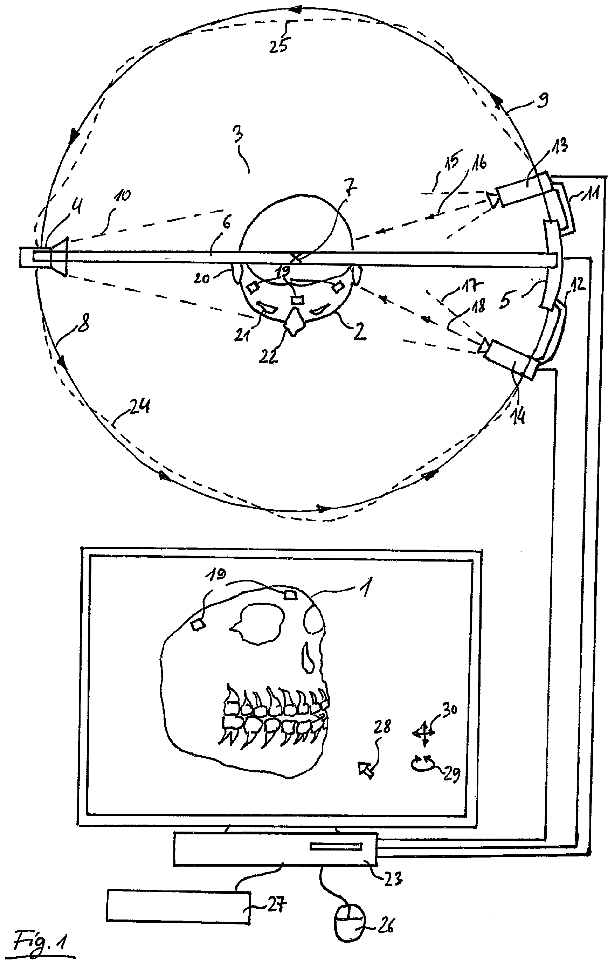

Die Erfindung wird anhand der Zeichnungen erläutert. Es zeigt, die
- Fig. 1: eine Skizze zur Verdeutlichung des Verfahrens zur Erzeugung einer dreidimensionalen Röntgenaufnahme.

### Ausführungsbeispiel

Die Fig. 1 zeigt eine Skizze zur Verdeutlichung des Verfahrens zur Erzeugung einer dreidimensionalen Röntgenaufnahme 1 eines Objekts 2, wie eines Patientenkopfes, mittels einer Röntgenvorrichtung 3. Die Röntgenvorrichtung 3 umfasst eine Röntgenquelle 4 und ein Röntgendetektor 5, die einen gegenüberliegenden Enden eines Tragarms 6 angeordnet sind. Der Tragarm 6 ist in der Röntgenvorrichtung 3 so gelagert, dass der Tragarm 6 mit der Röntgenquelle 4 und dem Röntgendetektor 5 um eine gemeinsame Drehachse 7, die als ein Kreuz dargestellt ist, rotiert. Während eines zumindest teilweisen Umlaufs wird die Röntgenquelle 4 nach der Aufnahmeplanung entlang einer ersten Soll-Trajektorie 8, die als eine durchgezogene Linie mit Pfeilen dargestellt ist, und der Röntgendetektor 5 entlang einer zweiten Soll-Trajektorie 9 rotiert. Im vorliegenden Ausführungsbeispiel ist die erste Soll-Trajektorie 8 und die zweite Soll-Trajektorie 9 kreisförmig. Die Röntgenquelle 4 erzeugt Röntgenstrahlen 10, die das Objekt 2 durchstrahlen und während des Umlaufs mittels des Röntgendetektors 5 erfasst werden. Auf diese Weise werden aus verschiedenen Aufnahmerichtungen zweidimensionale Röntgenaufnahmen des Objekts 2 erzeugt, die anschließend zur Berechnung der dreidimensionalen Röntgenaufnahme 1 durch Rekonstruktion verwendet werden. Am Röntgendetektor 5 sind zusätzlich über die Halterungen 11 und 12 eine erste optische Kamera 13 und eine zweite optische Kamera 14 angebracht. Die optischen Kameras 13 und 14 können alternativ auch unmittelbar am Tragarm 6 oder an der Röntgenquelle 4 angebracht sein. Die optischen Kameras 13 und 14 sind herkömmliche optische digitale Videokameras. Die erste optische Kamera 13 nimmt während des Umlaufs eine erste optische Aufnahme 15 des Objekts 2 aus einer ersten Aufnahmerichtung 16 auf, wobei die zweite optische Kamera 14 eine zweite optische Aufnahme 17 aus einer zweiten Aufnahmerichtung 18 aufnimmt. Die erste Aufnahmerichtung 16 und die zweite Aufnahmerichtung 18 der beiden optischen Kameras 13 und 14 bilden einen Winkel von etwa 30° zueinander, so dass die Lageänderung des Objekts 2 relativ zum Tragarm 6 damit relativ zu den daran angebrachten Röntgenquelle 4 und dem Röntgendetektor 5 bestimmt werden kann. Für die Bestimmung der Lagebeziehung des Objekts 2 relativ zum Tragarm 6 werden optische Markierungen 19 verwendet, die im Stirnbereich des Patientenkopfes 2 aufgeklebt werden. Im vorliegenden Ausführungsbeispiel sind die optischen Markierungen 19 rechteckig ausgeführt und können mittels der optischen Kameras 13 und 14 erfasst werden, um die genaue Lagebeziehung zwischen dem Patientenkopf 2 und dem Tragarm 6 bzw. den Röntgendetektor 5 zu bestimmen. Alternativ oder ergänzend dazu können zur Bestimmung der Lagebeziehung charakteristische Strukturen des Patientenkopfes 2, wie die Ohren 20, die Augen 21 und die Nase 22 verwendet werden. Die Markierungen 19 sowie die charakteristischen Strukturen 20, 21 und 22 können unter Anwendung von bekannten Mustererkennungsalgorithmen in den optischen Aufnahmen 15 und 17 erkannt werden. Die Bilddaten der beiden optischen Kameras 13 und 14 sowie die Bilddaten des Röntgendetektors 5 werden an einen Computer 23 übermittelt, wobei der Computer 23 die Berechnung der dreidimensionalen Röntgenaufnahme 1 durch Rekonstruktion aus den einzelnen zweidimensionalen Röntgenaufnahmen aus unterschiedlichen Aufnahmerichtungen durchführt, wobei die beiden optischen Aufnahmen 15 und 17 während des Umlaufs aus den verschiedenen Aufnahmerichtungen 16 und 18 verwendet werden, um eine Korrektur durchzuführen. Bei diesen Korrekturverfahren wird die gemessene Lagebeziehung zwischen Röntgendetektor 5 und dem Objekt 2 berücksichtigt, so dass Aufnahmefehler korrigiert werden, die durch eine Deformation des Tragarms 6 während des Umlauf-und/oder durch eine Lageänderung des Objekts 2 während des Umlaufs relativ zum Tragarm 6 verursacht werden. Dieses Korrekturverfahren kann mittels des Computers 23 in der verwendeten Software dadurch erfolgen, dass zur Berechnung der dreidimensionalen Röntgenaufnahme anstatt der geplanten Soll-Trajektorien 8 und 9 eine erste Ist-Bewegungstrajektorie 24 der Röntgenquelle 4 und eine zweite Ist- Bewegungstrajektorie 25 des Röntgendetektors 5 verwendet werden, die gestrichelt dargestellt sind. Die erste Ist-Trajektorie 24 und die zweite Ist-Trajektorie 25 sind zur Verdeutlichung skizzenhaft dargestellt. Die beiden Ist-Bewegungstrajektorien 24 und 25 sind so geformt, dass sie die Deformation des Tragarms 6 und die relative Lageänderung des Objekts 2 während des Umlaufs berücksichtigen. Dadurch kann dieselbe Software, wie bei einer herkömmlichen Röntgenvorrichtung, verwendet werden, wobei lediglich die Soll-Trajektorien 8 und 9 durch die korrigierten Ist-Trajektorien 24 und 25 ersetzt werden. Der Benutzer kann unter Verwendung von Eingabemitteln, wie einer Maus 26 und einer Tastatur 27 über einen virtuellen Cursor 28 in der dreidimensionalen Röntgenaufnahme 1 navigieren. Dabei kann der Benutzer ein virtuelles Navigationswerkzeug 29 zum Drehen der dreidimensionalen Röntgenaufnahme 1 und ein zweites virtuelles Navigationswerkzeug 30 zum Verschieben dreidimensionaler Röntgenaufnahme 1 verwenden. Die Markierungen 19 können auch Röntgensensitiv sein, so dass sie in der dreidimensionalen Röntgenaufnahme 1 sichtbar sind.

Alternativ dazu kann die Röntgenquelle 4 und der Röntgendetektor 5 mittels unterschiedlicher Antriebsmittel unabhängig voneinander entlang beliebiger Bewegungstrajektorien bewegt werden, die unter Verwendung einer ersten optischen Kamera an der Röntgenquelle und einer zweiten optischen Kamera am Röntgendetektor ermittelt werden.

### Bezugszeichen

- 1: Röntgenaufnahme
- 2: Objekt
- 3: Röntgenvorrichtung
- 4: Röntgenquelle
- 5: Röntgendetektor
- 6: Tragarm
- 7: Drehachse
- 8: erste Soll-Trajektorie
- 9: zweite Soll-Trajektorie
- 10: Röntgenstrahlen
- 11: Halterung
- 12: Halterung
- 13: erste Kamera
- 14: zweite Kamera
- 15: erste Aufnahme
- 16: erste Aufnahmerichtung
- 17: zweite Aufnahme
- 18: zweite Aufnahmerichtung
- 19: Markierung
- 20: Ohren
- 21: Augen
- 22: Nase
- 23: Computer
- 24: erste Ist-Trajektorie
- 25: zweite Ist-Trajektorie
- 26: Maus
- 27: Tastatur
- 28: Cursor
- 29: erstes Navigationswerkzeug
- 30: zweites Navigationswerkzeug

## Patentansprüche

1. Verfahren zur Erzeugung einer Röntgenaufnahme (1) eines Objekts (2) mittels einer Röntgenvorrichtung (3), wobei während eines zumindest teilweisen Umlaufs (8, 9) eine Röntgenquelle (4) und ein Röntgendetektor (5) um das Objekt (2) bewegt werden, wobei die von der Röntgenquelle (4) erzeugten und das Objekt(2) durchlaufenden Röntgenstrahlen (10) aus mehreren verschiedenen Richtungen mittels des Röntgendetektors (5) erfasst werden, wobei mindestens eine optische Kamera (13, 14) während des Umlaufs das Objekt (2) aufnimmt und mindestens eine erste optische Aufnahme (15, 17) erzeugt wird, wobei die mindestens eine optische Kamera (13, 14) eine bekannte und definierte Lagebeziehung zur Röntgenquelle (4) und/oder zum Röntgendetektor (5) aufweist, wobei die mindestens eine erste optische Aufnahme (15, 17) verwendet wird, um eine Bewegungstrajektorie (24, 25) des Objektes (2) relativ zur Röntgenquelle (4) und/oder zum Röntgendetektor (5) während des Umlaufs zu bestimmen, wobei die Röntgenquelle (4) und der Röntgendetektor (5) an einem Tragarm (6) angebracht sind; **dadurch gekennzeichnet, dass** die mindestens eine optische Kamera (13,14) am Röntgendetektor (5) fest angebracht ist, und dass die erste optische Aufnahme (15, 17) verwendet wird, um zusätzlich eine Deformation des Tragarms (6) während des Umlaufs zu bestimmen.

2. Verfahren nach Anspruch 1, **dadurch gekennzeichnet, dass** die ermittelte Bewegungstrajektorie des Objektes relativ zur Röntgenquelle (4) und/oder zum Röntgendetektor (5) verwendet wird, um eine dreidimensionale Röntgenaufnahme (1) aus mehreren zweidimensionalen Röntgenaufnahmen, die aus den unterschiedlichen Richtungen mittels des Röntgendetektors (5) erfasst wurden, zu berechnen.

3. Verfahren nach einem der Ansprüche 1 oder 2, **dadurch gekennzeichnet, dass** zusätzlich zur ersten optischen Kamera (13) eine zweite optische Kamera (14) das Objekt (2) während des Umlaufs aufnimmt und dabei eine zweite optische Aufnahme (17) erzeugt wird, wobei beide optischen Kameras (13, 14) eine bekannte und definierte Lagebeziehung zueinander aufweisen.

4. Verfahren nach einem der Ansprüche 1 bis 3, **dadurch gekennzeichnet, dass** am Objekt (2) optische Markierungen (19) angebracht sind, die mittels der optischen Kamera (13, 14) erfasst werden können.

5. Verfahren nach einem der Ansprüche 1 bis 4, **dadurch gekennzeichnet, dass** markante Strukturen (20, 21, 22) des Objekts (2) erfasst werden, um die Lagebeziehung des Objekts (2) relativ zur Röntgenquelle (4) und/oder zum Röntgendetektor (5) zu bestimmen.

6. Verfahren nach Anspruch 4 oder 5, **dadurch gekennzeichnet, dass** die optischen Markierungen (19) am Objekt (2) und /oder die markanten Strukturen (20, 21, 22) des Objekts (2) unter Verwendung von Mustererkennungsalgorithmen in der mindestens einen ersten optischen Aufnahme (15, 17) erkannt werden.

## Claims

1. A method for producing an X-ray image (1) of an object (2) by means of an X-ray device (3), wherein an X-ray source (4) and an X-ray detector (5) are moved around the object (2) during an at least partial rotation (8, 9), wherein the X-rays (10) generated by the X-ray source (4) and passing through the object (2) are recorded from a plurality of different directions by means of the X-ray detector (5), wherein at least one optical camera (13, 14) records the object (2) during the rotation and at least one first optical image (15, 17) is generated, wherein the at least one optical camera (13, 14) has a known and defined positional relationship to the X-ray source (4) and/or to the X-ray detector (5), wherein the at least one first optical image (15, 17) is used to determine a movement trajectory (24, 25) of the object (2) relative to the X-ray source (4) and/or to the X-ray detector (5) during the rotation, wherein the X-ray source (4) and the X-ray detector (5) are attached to a support arm (6); **characterised in that** the at least one optical camera (13, 14) is permanently attached to the X-ray detector (5), and **in that** the first optical image (15, 17) is used additionally to determine a deformation of the support arm (6) during the rotation.

2. The method according to claim 1, **characterised in that** the determined movement trajectory of the object relative to the X-ray source (4) and/or to the X-ray detector (5) is used to calculate a three-dimensional X-ray image (1) from a plurality of two-dimensional X-ray images recorded from different directions by means of the X-ray detector (5).

3. The method according to one of claims 1 or 2, **characterised in that**, in addition to the first optical camera (13), a second optical camera (14) records the object (2) during the rotation and a second optical image (17) is generated in the process, wherein both optical cameras (13, 14) have a known and defined positional relationship to one another.

4. The method according to one of claims 1 to 3, **characterised in that** optical markings (19) which can be recorded by means of the optical camera (13, 14) are attached to the object (2).

5. The method according to one of claims 1 to 4, **characterised in that** distinctive structures (20, 21, 22) of the object (2) are recorded in order to determine the positional relationship of the object (2) relative to the X-ray source (4) and/or to the X-ray detector (5).

6. The method according to claim 4 or 5, **characterised in that** the optical markings (19) on the object (2) and/or the distinctive structures (20, 21, 22) of the object (2) are recognised using pattern recognition algorithms in the at least one first optical image (15, 17).

## Revendications

1. Procédé pour la production d'une radiographie (1) d'un objet (2) au moyen d'un dispositif radiographique (3), dans lequel pendant une rotation au moins partielle (8, 9) une source de rayons X (4) et un détecteur de rayons X (5) se déplacent autour de l'objet (2), , les rayons X (10) générés par la source de rayons X (4) et traversant l'objet (2) étant détectés depuis plusieurs directions différentes par le détecteur de rayons X (5), au moins une caméra optique (13, 14) enregistrant l'objet (2) pendant la rotation de et au moins un premier enregistrement optique (15, 17) étant généré, ladite au moins une caméra optique (13, 14) ayant une relation de position connue et définie par rapport à la source de rayons X (4) et/ou par rapport au détecteur de rayons X (5), ledit au moins un premier enregistrement optique (15, 17) étant utilisé pour déterminer une trajectoire de déplacement (24, 25) de l'objet (2) par rapport à la source de rayons X (4) et/ou au détecteur de rayons X (5) pendant la rotation, la source de rayons X (4) et le détecteur de rayons X (5) étant fixés à un bras support (6) ; **caractérisé en ce que** ladite au moins une caméra optique (13, 14) est fermement fixée au détecteur de rayons X (5), et **en ce que** le premier enregistrement optique (15, 17) est utilisé pour déterminer en plus une déformation du bras support (6) pendant la rotation.

2. Procédé selon la revendication 1, **caractérisé en ce que** la trajectoire déterminée du déplacement de l'objet par rapport à la source de rayons X (4) et/ou au détecteur de rayons X (5) est utilisée pour calculer une radiographie (1) tridimensionnelle à partir de plusieurs radiographies bidimensionnelles prises depuis différentes directions au moyen du détecteur de rayons X (5).

3. Procédé selon l'une des revendications 1 ou 2, **caractérisé en ce que**, en plus de la première caméra optique (13), une deuxième caméra optique (14) enregistre l'objet (2) pendant la rotation et un deuxième enregistrement optique (17) est ce faisant généré, les deux caméras optiques (13, 14) ayant une relation de position connue et définie l'une par rapport à l'autre.

4. Procédé selon l'une quelconque des revendications 1 à 3, **caractérisé en ce que** des marquages optiques (19) détectables au moyen de la caméra optique (13, 14) sont apposés sur l'objet (2).

5. Procédé selon l'une quelconque des revendications 1 à 4, **caractérisé en ce que** des structures distinctives (20, 21, 22) de l'objet (2) sont enregistrées pour déterminer la relation de position de l'objet (2) par rapport à la source de rayons X (4) et/ou au détecteur de rayons X (5).

6. Procédé selon la revendication 4 ou 5, **caractérisé en ce que** les marquages optiques (19) sur l'objet (2) et/ou les structures distinctives (20, 21, 22) de l'objet (2) sont détectés dans au moins ledit premier enregistrement optique (15, 17) en utilisant des algorithmes de reconnaissance de forme.
